# EUROPÄISCHE PATENTANMELDUNG

(11) **EP 1 250 894 A1**
(43) Veröffentlichungstag der Anmeldung: **23.10.2002**
(21) Anmeldenummer: 02008810.0
(22) Anmeldetag: 19.04.2002
(51) Int. Cl.: A61C 1/00, G05D 11/02, A61L 2/24, A61L 2/18

(54) **Dosiervorrichtung zur Zugabe von Desinfektionsmitteln**

(30) Priorität: 19.04.2001 AT 6312001
(71) Anmelder: Konzett, Alfred, 6082 Patsch (AT); Pregenzer, Bruno, 6414 Mieming (AT)
(72) Erfinder: Konzett, Alfred, 6082 Patsch (AT); Pregenzer, Bruno, 6414 Mieming (AT)
(74) Vertreter: Torggler, Paul Norbert, Dr.

(57) **Zusammenfassung**

Eine Dosiervorrichtung zur Zugabe von Desinfektionsmittel in eine Versorgungseinrichtung für keimfreies Wasser, insbesondere an dentalen Arbeitsplätzen, weist einen Vorratsbehälter (6) für das Desinfektionsmittel, eine Zugabeeinrichtung (5) für das Desinfektionsmittel, und eine Steuereinrichtung (12) zur Betätigung der Zugabeeinrichtung (5) auf. Die Steuereinrichtung wird von einem in die Wasserleitung (1) eingesetzten Durchflußmengenmesser (2) angesteuert. Die Zugabeeinrichtung (5) umfaßt eine Pumpe, die mit einem Arbeitsmedium beaufschlagbar und durch Federkräfte rückstellbar ist.

## Beschreibung

Die Erfindung betrifft eine Dosiervorrichtung zur Zugabe von Desinfektionsmittel in eine Versorgungseinrichtung für keimfreies Wasser, insbesondere an dentalen Arbeitsplätzen, mit einem Vorratsbehälter für das Desinfektionsmittel, mit einer Zugabeeinrichtung für das Desinfektionsmittel, und mit einer Steuereinrichtung zur Betätigung der Zugabeeinrichtung, die von einem in die Wasserleitung eingesetzten Durchflußmengenmesser angesteuert wird.

An medizinischen, insbesondere zahnmedizinischen Behandlungs- oder Arbeitsplätzen ist eine Versorgung mit keimfreien Wasser unbedingt erforderlich. Es sind daher bereits eine Reihe von Lösungen vorgeschlagen worden, das üblicherweise aus dem öffentlichen Wasserleitungsnetz zu entnehmende Wasser mit chemischen Zusatzstoffen zu versehen, die das Wasser entkeimen, sodaß in der Zuleitung zum Behandlungs- oder Arbeitsplatz ein keimfreies Wasser-Zusatzstoffgemisch in einer vorbestimmten Konzentration zur Verfügung steht. Beispiele für bekannte Vorschläge finden sich in der DE 34 03 640 A sowie in der WO 98/13074 A, die eine Dosiervorrichtung der eingangs genannten Art zeigt. Bei dieser wird flüssiges Zusatzmittel im Vorratsbehälter mit Druckluft beaufschlagt und die Einspeisung in die Wasserleitung erfolgt über ein oder zwei elektrisch betätigbare Absperrventile, die entsprechend der Vorgaben des Durchflußmengenmessers angesteuert werden. Da der Wasserdruck in der Wasserleitung bei zumindest 2 bar liegen muß, um bestimmte Geräte am Arbeitsplatz verwenden bzw. bedienen zu können, ist dieser Leitungsdruck durch die Preßluftbeaufschlagung zu überwinden, d.h. der Vorratsbehälter muß höher druckfest ausgebildet sein und den hiefür geltenden Vorschriften entsprechen.

Nach der DE 34 03 640 A wird eine Hubkolbenpumpe verwendet, um den chemischen Zusatzstoff in entsprechender Dosierung in die Wasserleitung einzuspeisen, die an dieser Stelle eine Mischkammer umfaßt, um eine rasche und vollständige Durchmischung zu erzielen. Die Hubkolbenpumpe wird von einem Elektromotor mittels einer Nockenscheibe angetrieben. Der zur Überwindung des Wasserleitungsdrucks erforderliche Druck wird somit in der Hubkolbenpumpe bereitgestellt, und ein druckfester Vorratsbehälter erübrigt sich.

Da druckfeste Vorratsbehälter einerseits und elektromechanisch betätigte Hubkolbenpumpen andererseits relativ teure Bauelemente einer Dosiervorrichtungen darstellen, hat es sich die Erfindung zur Aufgabe gestellt, eine Dosiervorrichtung der eingangs genannten Art zu schaffen, die vor allem den Vorteil einer kostengünstigen Herstellung aufweist.

Erfindungsgemäß wird dies dadurch gelöst, daß die Zugabeeinrichtung eine Pumpe umfaßt, die mit einem Arbeitsmedium beaufschlagbar und durch Federkräfte rückstellbar ist. Auf diese Weise enthält die Dosiervorrichtung nur preisgünstige Bauelemente, d.h. es können einfache Vorratsbehälter, bevorzugt Verpackungsbeutel des chemischen Zusatzstoffes verwendet werden, und anstelle eines Elektromotors und der Nockensteuerung kann ein einfaches Magnetventil eingesetzt werden, das das Arbeitsmedium, insbesondere Wasser oder Luft, das ohnedies am Arbeitsplatz vorhanden ist, entsprechend der gewünschten Fördermenge der Pumpe zuführt.

Eine bevorzugte Ausführung sieht vor, daß die Zugabeeinrichtung zumindest zwei Membranpumpen aufweist, die alternierend mit einem Arbeitsmedium beaufschlagbar und durch Federkräfte rückstellbar sind. Die parallele Anordnung von zwei alternierend bzw. von mehr als zwei nicht gleichzeitig arbeitenden Membranpumpen ergibt trotz des abgesetzten Ausstoßes jeder einzelnen Pumpe eine annähernd gleichmäßige Zufuhr des Zusatzstoffes, solange die Pumpengarnitur angesteuert wird. Ansteuerungszeiten und -intervalle ergeben sich aus der erfaßten Durchflußmenge des Wassers und richten sich nach dem Verbrauch der angeschlossenen Geräte.

Ein annähernd konstanter Druck an den Abgabestellen für das Wasser-Zusatzstoffgemisch läßt sich in bevorzugter Ausführung dadurch erzielen, daß die Wasserleitung eine mit einem Druckmedium beaufschlagbare Misch- und Druckausgleichskammer enthält, in die die Zugabeeinrichtung mündet. Die Misch- und Ausgleichskammer kann flexible Wandungen aufweisen, und in einem Behälter angeordnet sein, der mit dem Druckmedium beaufschlagbar ist.

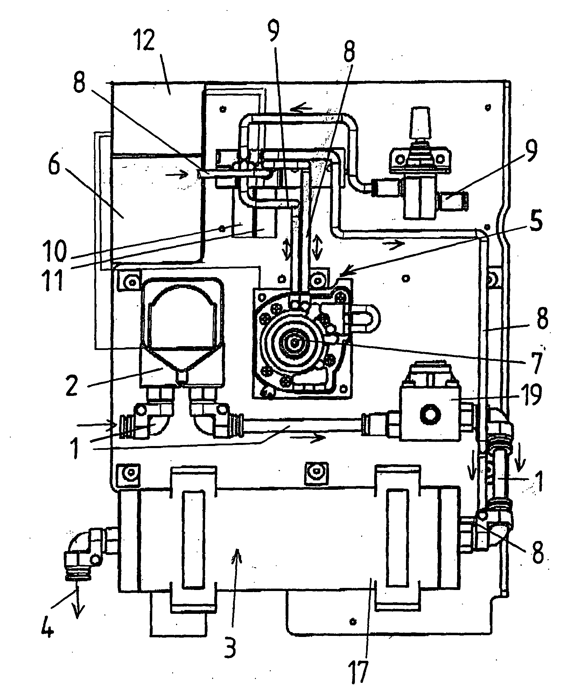

Nachstehend wird nun die Erfindung an Hand der Figur der beiliegenden Zeichnung näher beschrieben, die ein Schema der Dosiervorrichtung zeigt.

In der Schemadarstellung ist die Wasserleitung 1, die im allgemeinen mit dem Frischwassernetz verbunden wird, mit einem Durchflußmengenmesser 2 versehen. Stromabwärts des Durchflußmengenmessers 2 ist in einem Behälter 17 eine Mischkammer 3 vorgesehen, von der die Leitung zu einer oder mehreren Abgabestellen 4 weiterführt, die beispielsweise dentale Behandlungsgeräte, Speischalenauslaß od. dgl. sind. In die Mischkammer 3 mündet weiters eine Leitung 8, über die ein chemischer Zusatzstoff, beispielsweise ein flüssiges Desinfektionsmittel aus einem Vorratsbehälter 6 zugeführt wird. Die Zugabe des Mittels erfolgt mittels einer Zugabeeinrichtung 5, die eine mit einem Magnetventil 11 versehene Ansaugstrecke aus dem Vorratsbehälter 6, eine Pumpe 7 und die Leitung 8 umfaßt, wobei die Steuerung der Pumpe 7 über eine Steuereinrichtung 12 erfolgt. Diese berechnet aus der vom Durchflußmengenmesser 2 angegebenen Wasserverbrauchsmenge der Abgabestelle(n) 4 und der vorgegebenen Konzentration die Menge des aus dem Vorratsbehälter 6 zuzuführenden Zusatzstoffes. Als Pumpe 7 wird insbesondere eine Membranpumpe verwendet, die mit einem Arbeitsmedium, beispielsweise Preßluft, über die Leitung 9 beaufschlagt wird. In der Preßluftleitung 9 ist ein Magnetventil 10 vorgesehen, das ebenso wie das Magnetventil 11 von der Steuereinrichtung 12 betätigt wird.

Anstelle der gezeigten einzelnen Membranpumpe können auch zwei oder drei parallel angeordnete Pumpen vorgesehen werden, die nicht gleichzeitig arbeiten, um eine annähernd konstante Zuführung des Zusatzstoffs in die Mischkammer 3 zu bewirken. Die Mischkammer 3 kann zusätzlich auch als Druckausgleichskammer ausgeführt sein, um an der Abgabestelle 4 einen annähernd konstanten Druck zur Verfügung zu stellen. In diesem Fall weist die Mischkammer 3 bevorzugt eine flexible Wandung auf und ist in einem druckfesten Behälter 17 angeordnet, der mit einem Druckmedium, insbesondere ebenfalls Preßluft, beaufschlagt wird. In der Wasserleitung 1 kann weiters auch ein Druckregler 19 vorgesehen sein.

## Patentansprüche

1. Dosiervorrichtung zur Zugabe von Desinfektionsmittel in eine Versorgungseinrichtung für keimfreies Wasser, insbesondere an dentalen Arbeitsplätzen, mit einem Vorratsbehälter (6) für das Desinfektionsmittel, mit einer Zugabeeinrichtung (5) für das Desinfektionsmittel, und mit einer Steuereinrichtung (12) zur Betätigung der Zugabeeinrichtung (5), die von einem in die Wasserleitung (1) eingesetzten Durchflußmengenmesser (2) angesteuert wird, **dadurch gekennzeichnet, daß** die Zugabeeinrichtung (5) eine Pumpe umfaßt, die mit einem Arbeitsmedium beaufschlagbar und durch Federkräfte rückstellbar ist.

2. Dosiervorrichtung nach Anspruch 1, **dadurch gekennzeichnet, daß** die Zugabeeinrichtung (5) zumindest zwei Membranpumpen (7) aufweist, die alternierend mit einem Arbeitsmedium beaufschlagbar und durch Federkräfte rückstellbar sind.

3. Dosiervorrichtung nach Anspruch 1 oder 2, **dadurch gekennzeichnet, daß** die Wasserleitung (1) eine mit einem Druckmedium beaufschlagbare Misch- und Druckausgleichskammer (3) enthält, in die die Zugabeeinrichtung (5) mündet.

4. Dosiervorrichtung nach Anspruch 3, **dadurch gekennzeichnet**, die Misch- und Druckausgleichskammer (5) flexible Wandungen aufweist, und in einem mit dem Druckmedium beaufschlagbaren Behälter (17) angeordnet ist.
